# FASCICULE DE BREVET EUROPEEN

(11) **EP 0 638 550 B1**
(45) Date de publication et mention de la délivrance du brevet: **07.01.1998**
(21) Numéro de dépôt: 94401708.6
(22) Date de dépôt: 26.07.1994
(51) Int. Cl.: C07C 319/28, C07C 323/52, C07C 323/58, C07C 323/25, A61K 7/09, A61K 7/155

(54) **Procédé d'extraction des composés malodorants présents dans une formulation contenant au moins un composé comportant un groupe thiol et compositions désodorisées ainsi obtenues**
Verfahren zur Entfernung von übelriechenden Verbindungen aus einer Formulierung, die mindestens eine Verbindung mit einer Thiolgruppe enthält sowie sie enthaltende deodorisierte Zusammensetzungen
Method for extracting malodorous compounds present in a formulation containing at least one compound with a thiol group and deodorised compositions thus obtained

(30) Priorité: 02.08.1993 FR 9309486
(43) Date de publication de la demande: 15.02.1995
(73) Titulaire: L'OREAL, 75008 Paris (FR)
(72) Inventeur: Bauer, Daniel, F-93340 Le Raincy (FR); Richard, Françoise, F-93100 Montreuil Sous Bois (FR); Hassoun, Muriel, F-93320 Villepinte (FR); Malle, Gérard, F-77580 Villiers Sur Morin (FR); Samain, Henri, F-91570 Bievres (FR)
(74) Mandataire: Peuscet, Jacques

(56) Documents cités:
- EP-A- 0 261 387
- FR-A- 2 675 046
- FR-A- 2 679 448
- PATENT ABSTRACTS OF JAPAN vol. 4, no. 101 (C-19) 19 Juillet 1980 & JP-A-55 064 569 (DENKI KAGAKU KOGYO) 15 Mai 1980

## Description

La présente invention concerne un procédé d'extraction des composés malodorants présents dans une formulation contenant au moins un composé porteur d'un groupe fonctionnel thiol (-SH) et les compositions désodorisées, notamment cosmétiques, obtenues par ce procédé.

Les composés organiques porteurs de groupe(s) fonctionnel(s) thiol sont des composés bien connus, qui ont des applications de plus en plus nombreuses. Une de ces applications est la déformation (frisage et défrisage) permanente des cheveux, qui consiste, dans un premier temps, à réaliser l'ouverture des liaisons disulfure (S-S) des unités cystine de la kératine à l'aide d'une composition contenant au moins un composé organique porteur de groupe fonctionnel thiol agissant comme réducteur (étape de réduction), ce qui permet de conférer aux cheveux la forme que l'on souhaite ; puis, après avoir rincé la chevelure, à reconstituer, dans un second temps, lesdites liaisons disulfure en appliquant, sur les cheveux, une composition oxydante (étape d'oxydation, dite aussi de fixation), de façon à fixer les cheveux dans la forme qui leur a été donnée ; dans ce but, on utilise particulièrement l'acide thioglycolique, l'acide thiolactique ou leurs mélanges, ou encore les esters de ces acides, par exemple les monothioglycolates de glycérol ou de glycol, ou aussi la cystéine ou la cystéamine. Les acides thioglycolique ou thiolactique et leurs sels sont également utilisés dans des laits et crèmes dépilatoires. Les acides thioglycolique et thiolactique ainsi que la cystéine sont aussi employés comme produits intermédiaires dans la fabrication de produits pharmaceutiques.

Malheureusement, si, en général, les composés porteurs d'un groupe fonctionnel thiol ont, à l'état pur, une odeur qui n'est pas désagréable, ils contiennent toujours, en pratique, des composés sulfurés tels que l'hydrogène sulfuré et des mercaptans de faible poids moléculaire, en particulier le méthanethiol ou l'éthanethiol, qui ont une odeur nauséabonde particulièrement désagréable. Il suffit de très faibles quantités de ces composés sulfurés pour que l'on perçoive leur présence à l'odorat, le nez étant, dans ce cas, le meilleur instrument de détection. Dans la suite de la description et dans les revendications, ces composés sulfurés seront désignés par le terme "composés malodorants".

La présence de ces composés malodorants est liée à divers processus mal connus de décomposition des composés porteurs d'un groupe thiol, en particulier, par oxydation.

Dans les différentes applications des composés porteurs d'un groupe fonctionnel thiol, et plus particulièrement dans leurs applications cosmétiques, l'odeur dégagée par les produits mis en oeuvre constitue une réelle gêne pour les utilisateurs. On a essayé de masquer l'odeur par des parfums mais, en général, l'odeur est trop puissante pour pouvoir être masquée de façon significative.

On a donc cherché à éliminer les composés malodorants. Dans ce but, on a déjà proposé, dans la demande de brevet japonais publiée sous le n° 84-027 866, de désodoriser l'acide thioglycolique, pur ou en solution aqueuse, par extraction à l'aide d'un hydrocarbure non aromatique en C₄-C₈. Etant donné que, pour effectuer l'extraction, il est nécessaire d'utiliser une installation relativement volumineuse, il est pratiquement impossible de désodoriser les composés porteurs de groupe fonctionnel thiol juste avant leur utilisation de sorte que leur stockage est, en pratique, obligatoire. Or, on a constaté que, si ce procédé permet d'obtenir un acide désodorisé, l'effet de désodorisation obtenu n'est pas durable car les composés malodorants se reforment au cours du stockage, en particulier en présence d'oxygène, et même, dans certains cas, l'odeur revient à un niveau supérieur au niveau initial.

Dans la demande de brevet japonais JP-A-55 064 569, on a décrit un procédé de purification de l'acide thioglycolique ; ce procédé est destiné à éliminer deux sous-produits de fabrication, à savoir, l'acide dithioglycolique (HOOC-CH₂-S-S-CH₂-COOH) et l'acide thiodiglycolique (S-(CH₂-CO₂H)₂), qui ne comportent pas de groupe -SH mais respectivement des liaisons S-S et S-C ; les deux sous-produits sont absorbés alors que l'acide thioglycolique reste dans la solution. Ce document ne permettait donc pas de séparer d'un côté un composé portant des groupes -SH tel que l'acide thioglycolique et de l'autre des composés malodorants généralement constitués de mercaptans qui portent aussi des groupes -SH.

On désirait donc un procédé qui permette, juste avant l'utilisation, d'éliminer les composés malodorants des formulations contenant au moins un composé porteur d'un groupe fonctionnel thiol sans qu'une installation volumineuse soit nécessaire.

Il est connu, par ailleurs, de dépolluer des compositions gazeuses ou en solution, en particulier d'éliminer H₂S et les mercaptans de faible poids moléculaire, par absorption physique sur un produit absorbant pulvérulent. Mais il est bien connu que ces produits absorbants sont le plus souvent chargés en oxygène. L'homme de métier avait donc toute raison de penser qu'en mettant en contact ces produits absorbants avec des composés porteurs d'un groupe fonctionnel thiol, la dégradation de ces composés par oxygénation avec formation de composés malodorants serait trop rapide, de sorte qu'il serait impossible, même pendant un temps limité, d'obtenir une composition désodorisée ou de conserver les propriétés, notamment réductrices, de cette composition.

Selon la présente invention, on a trouvé qu'en réalité, contrairement au préjugé de l'homme de métier, il était possible d'utiliser un agent absorbant pulvérulent non soluble dans la formulation contenant au moins un composé porteur d'un groupe fonctionnel thiol pour désodoriser efficacement ladite formulation, sans que les propriétés du composé porteur d'un groupe thiol ne soient dégradées de façon significative.

La présente invention a pour objet un procédé d'extraction des composés malodorants présents dans une formulation liquide contenant au moins un composé comportant un groupe fonctionnel thiol de formule :

HS_A_Y_B (I)

formule dans laquelle Y représente -COO- ou -NH- et
a) lorsque Y désigne -COO- :
   - A représente :
      - le radical divalent : -(CH₂)ₙ où n est 1 ou 2
      - le radical divalent : 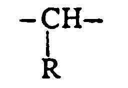 où R représente un radical alkyle, linéaire ou ramifié, en C₁-C₃ et
   - B représente les radicaux -H ; -CH₂-CH₂OH ; -CH₂-CH OH-CH₂ OH, 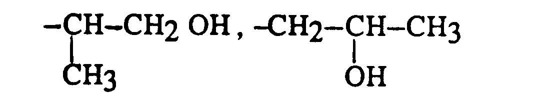
b) lorsque Y représente -NH-
   - A représente
      -CH₂-CH₂- ou 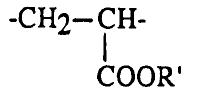 R' représentant H ou un radical méthyle ou éthyle
   - B représente -H ou, lorsque A représente -CH₂-CH₂-,
      B représente également un radical -CO-R'',
      R'' représentant un radical alkyle, linéaire ou ramifié, en C₁-C₄, et
caractérisé par le fait que l'on met en contact, avant son utilisation, ladite formulation avec un agent absorbant pulvérulent non soluble dans ladite formulation, ledit agent absorbant étant tel que, lorsqu'on le met en suspension dans une solution aqueuse contenant 92 g/l d'acide thioglycolique, ladite solution aqueuse contienne encore au moins 30 % en poids de l'acide thioglycolique initial après 15 minutes de contact.

La formulation liquide soumise au procédé selon l'invention peut comporter un composé de formule (I) pur ou un mélange de composés de formule (I) purs, lorsque ce(s) composé(s) est (sont) disponible(s) sous forme liquide, ou encore une solution contenant au moins un composé de formule (I), le(s) composé(s) de formule (I) pouvant être éventuellement associés à différents ingrédients de formulation selon leur utilisation. En outre, la formulation liquide peut se présenter sous forme de crème plus ou moins épaisse ou de gel plus ou moins rigide.

Le(s) composé(s) de formule (I) est (sont) avantageusement choisi(s) dans le groupe formé par l'acide thioglycolique (ou mercaptoacétique), l'acide thiolactique (ou acide mercapto-2 propionique), l'acide mercapto-3 propionique, le monothioglycolate de glycérol, le mercapto-2 propionate de glycérol, un mélange azéotrope de thioglycolate d'hydroxy-2 propyle et de thioglycolate d'hydroxy-2 méthyl-1 éthyle (tel que décrit dans FR-A 2 679 448), la cystéamine (ou amino-2 éthanethiol) et ses dérivés N-acylés, le radical acyle comportant de 2 à 5 atomes de carbone, la cystéine et les cystéinates de méthyle et d'éthyle. On préfère les acides thioglycolique et thiolactique.

L'agent absorbant contient, de préférence, au moins un produit absorbant choisi dans le groupe formé par :
- les charbons actifs, tels que ceux commercialisés sous les dénominations "PICACTIF CB 506", "PGV 120 EWN", "PMC 2" par la société "PICA", ceux commercialisés sous la dénomination "ACTICARBONE" par la "C.E.C.A." et ceux commercialisés sous les dénominations "NORIT CA 1", "NORIT CN 1", "NORIT SA 2", "NORIT SA 4", "NORIT SX 2", "NORIT SX ULTRA" et "NORIT W 20" par la société "NORIT",
- le charbon animal,
- le talc, le carbonate de calcium, la terre d'infusoires, les argiles, telles que les bentonites et les montmorillonites,
- la sciure de bois et les polymères organiques absorbants, tel que le polymère obtenu par polymérisation du 2,6-diphényl paraoxyphénylène commercialisé sous la dénomination "TENAX" par la société "INTERCHIM".

L'agent absorbant a, de préférence, une granulométrie comprise entre 0,1 et 100 µm.

L'agent absorbant et la formulation liquide sont mis en contact, peu de temps avant l'utilisation pour que la capacité réductrice de la formulation soit conservée de façon suffisante, mais néanmoins suffisamment longtemps avant l'utilisation pour que les composés malodorants aient le temps d'être absorbés ; on préfère que la mise en contact intervienne entre 5 minutes et 30 minutes avant l'utilisation.

Selon un premier mode de mise en oeuvre de l'invention, l'agent absorbant est mélangé avec la formulation liquide. Il faut noter que, selon ce mode de mise en oeuvre, on utilise, de préférence, directement la composition constituée par le mélange contenant la formulation et l'agent absorbant. Dans ce but, on peut conditionner séparément la formulation et l'agent absorbant et n'effectuer le mélange que peu avant l'utilisation de la composition.

Selon un second mode de mise en oeuvre, on fait passer la formulation liquide à travers une couche d'agent absorbant : on peut, par exemple, mettre en oeuvre dans ce but le dispositif décrit dans FR-A-2 597 443 et utiliser ensuite la composition désodorisée obtenue.

La présente invention a également pour objet la composition désodorisée obtenue par le procédé décrit ci-dessus.

La présente invention a plus particulièrement pour objet une composition de ce type contenant d'une part, une formulation renfermant au moins un composé de formule (I) et d'autre part, un agent absorbant pulvérulent mélangé à ladite formulation : cette composition est destinée à être appliquée sur un élément kératinique humain tel que la peau, les cheveux et les poils ; cette composition est, de préférence, une composition cosmétique pour la déformation des cheveux ou une composition dépilatoire.

Le(s) composé(s) de formule (I) est(sont) généralement présent(s) à une concentration comprise entre 2 et 30 %, et, de préférence, entre 5 et 25 % en poids par rapport au poids total de la composition et l'agent absorbant est présent à une concentration comprise entre 0,5 et 30 % en poids par rapport au poids total de la composition, et, de préférence, entre 1 et 10 % en poids par rapport au poids total de la composition.

Le pH de la composition est, de préférence, compris entre 5 et 12,5 et est obtenu à l'aide d'un agent alcalinisant, tel que, par exemple, l'ammoniaque, la monoéthanolamine, la diéthanolamine, la triéthanolamine, un carbonate ou bicarbonate alcalin ou d'ammonium, un hydroxyde alcalin, ou à l'aide d'un agent acidifiant tel que, par exemple, l'acide chlorhydrique, l'acide acétique, l'acide lactique, l'acide oxalique ou l'acide borique. Lorsque la composition est destinée à être utilisée pour la déformation permanente des cheveux, le pH est généralement compris entre 5 et 10 et lorsqu'elle est destinée à être utilisée pour la dépilation, le pH est généralement compris entre 9 et 12,5.

La composition peut, en outre, contenir divers ingrédients de formulation comme, par exemple, des polymères cationiques tels que ceux utilisés dans les compositions des brevets français n° 79-32078 et 80-26421 ou encore des polymères cationiques de type ionène tels que ceux utilisés dans les compositions du brevet français n° 82-17364, des agents adoucissants tels que les ammonium quaternaire dérivés de la lanoline, des hydrolysats de protéines, des cires, des agents opacifiants, des parfums, des colorants, des agents tensioactifs non-ioniques ou cationiques, des alcools tels que l'éthanol, le propanol, l'isopropanol, le propanediol-1,2, le butanediol-1,2 ou le glycérol, des agents traitants ou encore des agents de pénétration, tels que l'urée, la pyrrolidone ou la thiamorpholinone.

Le véhicule des compositions selon l'invention est, de préférence, de l'eau ou une solution hydroalcoolique d'un alcool inférieur tel que l'éthanol, l'isopropanol ou le butanol.

Il convient de constater que l'invention ci-dessus définie n'était aucunement suggérée par la demande de brevet japonais JP-A-55 064 569 mentionnée précédemment. En effet, il n'était pas évident, au vu de ce document, que tous les composés portant des groupes -SH ne se fixeraient pas sur l'absorbant et que, par conséquent, il serait possible de séparer des composés malodorants l'acide thioglycolique et les autres composés de formule HS-A-YB. Selon la présente invention, on prépare une composition cosmétique contenant à la fois de l'acide thioglycolique et un absorbant, cette composition étant totalement inodore. Au demeurant, ce résultat n'est obtenu qu'avec des absorbants répondant à une définition particulière qui n'est aucunement suggérée par la demande de brevet japonais précitée.

Lorsque les compositions sont destinées à une opération de défrisage ou de décrêpage des cheveux, la composition réductrice est, de préférence, sous forme d'une crème, de façon à maintenir les cheveux aussi raides que possible. On réalise ces crèmes sous forme d'émulsions, par exemple à base de stéarate de glycéryle, de stéarate de glycol, de cires auto-émulsionnables, d'alcools gras, etc... On peut également utiliser des liquides ou des gels contenant des agents épaississants tels que des polymères ou des copolymères carboxyvinyliques, qui maintiennent les cheveux dans la position lisse pendant le temps de pose.

Pour effectuer la déformation permanente des cheveux, dans une première étape, on réduit les liaisons disulfures de la kératine par application, pendant environ 5 à 60 minutes, d'une composition définie ci-dessus puis, dans une seconde étape, on reforme lesdites liaisons par application d'une composition oxydante ou éventuellement en laissant agir l'oxygène de l'air.

Pour effectuer une ondulation des cheveux, on applique une composition telle que définie ci-dessus sur des cheveux mouillés préalablement enroulés sur des rouleaux ayant de 4 à 20 mm de diamètre, la composition pouvant éventuellement être appliquée au fur et à mesure de l'enroulage des cheveux ; on laisse ensuite agir la composition réductrice pendant un temps de 5 à 60 minutes, de préférence de 5 à 30 minutes, puis on rince abondamment ; après quoi on applique, sur les cheveux enroulés, un agent de fixation permettant de reformer les liaisons disulfure de la kératine pendant un temps de pose compris entre 2 et 10 minutes. Après avoir enlevé les rouleaux, on rince abondamment la chevelure. L'agent de fixation couramment utilisé contient comme oxydant de l'eau oxygénée, un bromate alcalin, un persel, un polythionate ou un mélange de bromate alcalin et de persel. Le pH de l'agent de fixation est généralement compris entre 2 et 10. Cette oxydation peut être effectuée immédiatement ou être différée.

Pour effectuer un défrisage ou un décrêpage des cheveux, on applique sur les cheveux une composition selon l'invention puis on soumet les cheveux à une déformation mécanique permettant de les fixer dans leur nouvelle forme, par une opération de lissage des cheveux avec un peigne à larges dents, avec le dos d'un peigne ou à la main. Après un temps de pose de 5 à 60 minutes, en particulier de 5 à 30 minutes, on procède alors à un nouveau lissage puis on rince soigneusement et on applique un agent de fixation tel que défini ci-dessus, qu'on laisse agir pendant environ 2 à 10 minutes, puis on rince abondamment les cheveux.

Les exemples donnés ci-après, à titre illustratif et nullement limitatif, permettront de mieux comprendre l'invention.

### EXEMPLE 1

### I - Préparation

A - On prépare une formulation réductrice constituée d'une solution d'acide thioglycolique 1 M, dont on ajuste le pH à une valeur de 8,5 par addition de monoéthanolamine. On ajoute ensuite à la formulation obtenue 20 % en poids (par rapport au poids de ladite formulation) de charbon actif commercialisé sous la dénomination "PICACTIF CB 506" par la société "PICA". On obtient ainsi une composition A que l'on utilise, comme indiqué ci-après, dix minutes après sa préparation.
B - On a également préparé, à titre comparatif, une composition B identique sauf que l'on n'ajoute pas de charbon actif et on l'utilise également dix minutes après sa préparation.

### II - Essai

On a effectué avec les compositions A et B une permanente sur deux mèches de cheveux naturels identiques. Les compositions A et B ont été utilisées à raison de 2 g de composition pour 1 g de cheveux. Elles sont appliquées sur des cheveux mouillés préalablement enroulés sur des rouleaux. Après un temps de pose de 15 minutes, les cheveux ont été rincés puis fixés par une solution d'eau oxygénée à 8 volumes ayant un pH de 3, appliquée pendant 5 minutes, à raison de 2 g par gramme de cheveux, puis rincés à nouveau et séchés.

On a constaté que la frisure obtenue avec la composition A est identique à celle obtenue avec la composition B. Par conséquent, la présence de charbon actif dans la composition ne modifie pas le pouvoir réducteur de l'acide thioglycolique.

De plus, pendant la phase de réduction par la composition A, aucune mauvaise odeur n'a été perçue ; de même, après rinçage et fixation, on n'a pas remarqué d'odeurs désagréables. Par contre, avec la composition B, on a constaté le développement d'odeurs désagréables pendant la phase de réduction.

### EXEMPLE 2

### I - Préparation

C - On a préparé une solution de cystéamine 1 M et on a ajusté son pH à une valeur de 8,5 par addition de monoéthanolamine. On a ajouté à cette formulation 30 % en poids (par rapport au poids de la formulation) de charbon actif commercialisé sous la dénomination "PM C2" par la société "PICA". On a obtenu une composition C que l'on utilise dix minutes après sa préparation.
D - On a préparé simultanément, à titre comparatif, une solution D identique à la composition C, mais ne contenant pas de charbon actif et on l'utilise également dix minutes après sa préparation.

### II - Essai

On a effectué une permanente avec ces deux compositions C et D, de la même façon que dans l'exemple 1, sur des cheveux naturels chatains foncés.

On a constaté que, dans les deux cas, la frisure obtenue était identique et que, dans le cas de la composition C, il ne se formait pas d'odeurs désagréables lors de la phase de réduction, alors qu'il se formait des odeurs désagréables dans le cas de la composition D.

### EXEMPLE 3

### I-Préparation

E - On a préparé une solution de cystéine 1 M et on a ajusté son pH à une valeur de 8,5 par addition de monoéthanolamine. On a ajouté à cette formulation 15 % en poids (par rapport au poids de la formulation) d'un mélange 50/50 en poids de montmorillonite et de charbon actif commercialisé sous la dénomination "FGV 120 EWN" par la société "PICA". On obtient une composition E que l'on utilise, comme indiqué ci-après, dix minutes après sa préparation.
F - On a préparé, à titre comparatif, une composition F identique à la composition E, sauf qu'elle ne contient ni argile ni charbon actif et on l'utilise également dix minutes après sa préparation.

### II - Essai

On a effectué avec les compositions E et F une permanente sur cheveux naturels chatains foncés dans les mêmes conditions que dans l'exemple 1.

On a constaté que la frisure obtenue avec la composition E est identique à celle obtenue avec la composition F et que, dans la phase de réduction de la permanente, il ne formait pas d'odeurs désagréables avec la composition E, tandis qu'il apparaissait des odeurs désagréables avec la composition F.

## Revendications

1. Procédé d' extraction des composés malodorants présents dans une formulation liquide contenant au moins un composé porteur d'un groupe fonctionnel thiol de formule :
HS―A―Y―B (I)
formule dans laquelle Y représente -COO- ou -NH- et
a) lorsque Y désigne -COO- :
• A représente :
- le radical divalent : -(CH₂)ₙ où n est 1 ou 2
- le radical divalent : 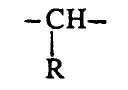 où R représente un radical alkyle, linéaire ou ramifié, en C₁-C₃ et
• B représente les radicaux -H ; -CH₂-CH₂OH ; -CH₂-CH OH-CH₂ OH, 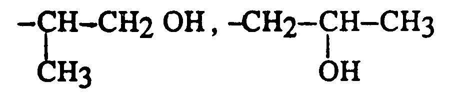
b) lorsque Y représente -NH-
• A représente
-CH₂-CH₂- ou 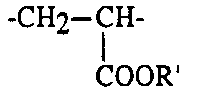 R' représentant H ou un radical méthyle ou éthyle
• B représente -H ou, lorsque A représente -CH₂-CH₂-,
B représente également un radical -CO-R'',
R'' représentant un radical alkyle, linéaire ou ramifié, en C₁-C₄, et
caractérisé par le fait que l'on met en contact ladite formulation avec un agent absorbant pulvérulent, pour obtenir une composition désodorisée, destinée à une utilisation ultérieure, ledit agent absorbant étant tel que, lorsqu'on le met en suspension dans une solution aqueuse contenant 92 g/l d'acide thioglycolique, ladite solution aqueuse contienne encore au moins 30 % en poids de l'acide thioglycolique initial après 15 minutes de contact.

2. Procédé selon la revendication 1, caractérisé par le fait que l'on choisit les composés de formule (I) dans le groupe formé par l'acide thioglycolique, l'acide thiolactique, l'acide mercapto-3 propionique, le monothioglycolate de glycérol, le mercapto-2 propionate de glycérol, un mélange azéotrope de thioglycolate d'hydroxy-2 propyle et de thioglycolate d'hydroxy-2 méthyl-1 éthyle, la cystéamine et ses dérivés N-acylés, le radical acyle comportant de 2 à 5 atomes de carbone, la cystéine et les cystéinates de méthyle et d'éthyle.

3. Procédé selon l'une des revendications 1 ou 2, caractérisé par le fait que l'agent absorbant comprend au moins un produit absorbant choisi dans le groupe formé par les charbons actifs, le charbon animal, le talc, le carbonate de calcium, la terre d'infusoires, les argiles, la sciure de bois et les polymères organiques absorbants.

4. Procédé selon l'une des revendications 1 à 3, caractérisé par le fait que l'agent absorbant a une granulométrie comprise entre 0,1 et 100 µm.

5. Procédé selon l'une des revendications 1 à 4, caractérisé par le fait que l'on met en contact la formulation liquide et l'agent absorbant par mélange.

6. Procédé selon la revendication 5, caractérisé par le fait que l'on met en contact la formulation avec l'agent absorbant entre 5 minutes et 30 minutes avant l'utilisation de la composition.

7. Procédé selon l'une des revendications 1 à 4, caractérisé par le fait qu'on fait passer la formulation liquide à travers une couche d'agent absorbant.

8. Composition désodorisée obtenue par le procédé selon une des revendications 1 à 4.

9. Composition cosmétique désodorisée, destinée à être appliquée sur un élément kératinique humain tel que la peau, les cheveux et les poils, caractérisé par le fait qu'elle contient au moins un composé de formule (I) tel que défini dans la revendication 1 et au moins un agent absorbant pulvérulent tel que défini dans la revendication 1.

10. Composition selon la revendication 9, caractérisée par le fait que le(s) composé(s) de formule (I) est (sont) présent(s) à une concentration comprise entre 2 et 30 % en poids par rapport au poids total de la composition et l'agent absorbant a une concentration comprise entre 0,5 et 30 % en poids par rapport au poids total de la composition.

11. Composition selon la revendication 10, caractérisée par le fait que le(s) composé(s) de formule (I) y est (sont) présent(s) à une concentration comprise entre 5 et 25 % en poids par rapport au poids total de la composition et l'agent absorbant à une concentration comprise entre 1 et 10 % en poids par rapport au poids total de la composition.

12. Composition selon l'une des revendications 9 à 11, caractérisée par le fait que son pH est compris entre 5 et 12,5.

13. Composition selon la revendication 12, caractérisée par le fait qu'elle comporte, pour l'ajustement de son pH, un agent alcalinisant choisi dans le groupe formé par : l'ammoniaque, la monoéthanolamine, la diéthanolamine, la triéthanolamine, les carbonates alcalins, les bicarbonates alcalins, le carbonate d'ammonium, le bicarbonate d'ammonium et un hydroxyde alcalin, ou un agent acidifiant choisi dans le groupe formé par l'acide chlorhydrique, l'acide acétique, l'acide lactique, l'acide oxalique et l'acide borique.

14. Composition selon l'une des revendications 12 ou 13 destinée à la déformation permanente des cheveux, caractérisée par le fait que son pH est compris entre 5 et 10.

15. Composition selon l'une des revendications 12 ou 13 destinée à la dépilation, caractérisée par le fait que son pH est compris entre 9 et 12,5.

16. Composition selon l'une des revendications 9 à 15, caractérisée par le fait qu'elle contient une solution aqueuse ou hydroalcoolique d'au moins un composé de formule (I).

## Claims

1. Method for extracting malodourous compounds present in a liquid formulation containing at least one compound bearing a thiol functional group of formula:
HS-A-Y-B (I)
in which formula Y represents -COO- or -NH- and
a) when Y denotes -COO- :
• A represents:
- the divalent radical: -(CH₂)ₙ where n is 1 or 2
- the divalent radical: 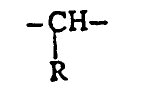 where R represents a linear or branched C₁-C₃ alkyl radical, and
• B represents the radicals -H; -CH₂-CH₂OH; -CH₂-CHOH-CH₂OH, 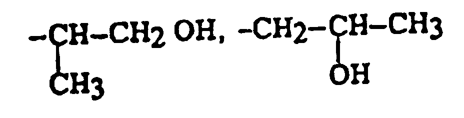
b) when Y represents -NH-
• A represents
-CH₂-CH₂- or 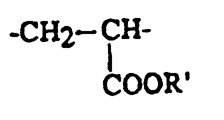 R' representing H or a methyl or ethyl radical
• B represents -H or, when A represents -CH₂-CH₂-,
B also represents a radical -CO-R'',
R'' representing a linear or branched C₁-C₄ alkyl radical, and
charcterized in that the said formulation is placed in contact with a pulverulent absorbing agent, in order to obtain a deodourized composition, intended for a subsequent use, the said absorbing agent being such that, when it is suspended in an aqueous solution containing 92 g/l of thioglycolic acid, the said aqueous solution also contains at least 30% by weight of the initial thioglycolic acid after contact for 15 minutes.

2. Method according to Claim 1, characterized in that the compounds of formula (I) are chosen from the group formed by thioglycolic acid, thiolactic acid, 3-mercaptopropionic acid, glyceryl monothioglycolate, glyceryl 2-mercaptopropionate, an azeotropic mixture of 2-hydroxypropyl thioglycolate and 2-hydroxy-1-methylethyl thioglycolate, cysteamine and the N-acyl derivatives thereof, the acyl radical containing from 2 to 5 carbon atoms, cysteine and methyl and ethyl cysteinates.

3. Method according to either of Claims 1 and 2, characterized in that the absorbing agent comprises at least one absorbent product chosen from the group formed by activated charcoals, animal charcoal, talc, calcium carbonate, diatomaceous earth, clays, wood sawdust and absorbent organic polymers.

4. Method according to one of Claims 1 to 3, characterized in that the absorbing agent has a particle size between 0.1 and 100 µm.

5. Method according to one of Claims 1 to 4, characterized in that the liquid formulation and the absorbing agent are placed in contact by mixing.

6. Method according to Claim 5, characterized in that the formulation is placed in contact with the absorbing agent between 5 minutes and 30 minutes before use of the composition.

7. Method according to one of Claims 1 to 4, characterized in that the liquid formulation is passed through a layer of absorbing agent.

8. Deodorized composition obtained by the method according to one of Claims 1 to 4.

9. Deodorized cosmetic composition intended to be applied to a human keratinous substance such as the skin, head hair and body hair, characterized in that it contains at least one compound of formula (I) as defined in Claim 1 and at least one pulverulent absorbing agent as defined in Claim 1.

10. Composition according to Claim 9, characterized in that the compound(s) of formula (I) is (are) present at a concentration between 2 and 30% by weight relative to the total weight of the composition and the absorbing agent at a concentration between 0.5 and 30% by weight relative to the total weight of the composition.

11. Composition according to Claim 10, characterized in that the compound(s) of formula (I) is (are) present therein at a concentration between 5 and 25% by weight relative to the total weight of the composition and the absorbing agent at a concentration between 1 and 10% by weight relative to the total weight of the composition.

12. Composition according to one of Claims 9 to 11, characterized in that its pH is between 5 and 12.5.

13. Composition according to Claim 12, characterized in that it contains, in order to adjust its pH, a basifying agent chosen from the group formed by: aqueous ammonia, monoethanolamine, diethanolamine, triethanolamine, alkali metal carbonates, alkali metal bicarbonates, ammonium carbonate, ammonium bicarbonate and an alkali metal hydroxide, or an acidifying agent chosen from the group formed by hydrochloric acid, acetic acid, lactic acid, oxalic acid and boric acid.

14. Composition according to either of Claims 12 and 13 which is intended for the permanent reshaping of hair, characterized in that its pH is between 5 and 10.

15. Composition according to either of Claims 12 and 13 which is intended for hair removal, characterized in that its pH is between 9 and 12.5.

16. Composition according to one of Claims 9 to 15, characterized in that it contains an aqueous or aqueous-alcoholic solution of at least one compound of formula (I).

## Patentansprüche

1. Verfahren zur Entfernung von übelriechenden Verbindungen aus einer flüssigen Formulierung, die mindestens eine Verbindung mit einer Thiolgruppe der Formel
HS-A-Y-B (I)
enthält, in welcher Y für -COO- oder -NH- steht und
a) wenn Y für -COO- steht,
• A folgendes darstellt:
- das zweiwertige Radikal -(CH₂)ₙ, in dem n 1 oder 2 ist,
- das zweiwertige Radikal 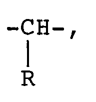 in dem R ein geradkettiges oder verzweigtes C₁-C₃-Alkyl-Radikal ist, und
• B die folgenden Radikale darstellt: -H; -CH₂-CH₂OH; -CH₂-CHOH-CH₂OH, 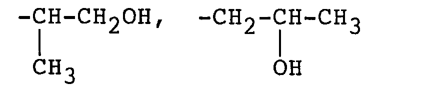
b) wenn Y für -NH- steht,
• A für -CH₂-CH₂- oder 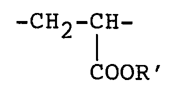 steht, worin R' für H oder ein Methyl- oder Ethyl-radikal steht,
• B für -H steht oder, wenn A für -CH₂-CH₂- steht,
B auch ein -CO-R'' -Radikal darstellt,
worin R'' ein geradkettiges oder verzweigtes C₁-C₄-Alkyl-Radikal darstellt,
dadurch gekennzeichnet, daß man diese Formulierung mit einem pulverförmigen Absorptionsmittel in Kontakt bringt, um eine zur weiteren Verwendung bestimmte desodorierte Zusammensetzung zu erhalten, wobei das Absorptionsmittel so beschaffen ist, daß, wenn man es in einer 92 g/l Thioglykolsäure enthaltenen wässrigen Lösung in Suspension bringt, diese wässrige Lösung nach 15 Minuten Kontakt noch mindestens 30 Gew.-% der ursprünglichen Thioglycolsäure enthält.

2. Verfahren nach Anspruch 1, dadurch gekennzeichnet, daß man die Verbindungen der Formel (I) in der Gruppe auswählt, die aus folgendem besteht: Thioglycolsäure, Thiomilchsäure, 3-Mercapto-Propionsäure, Glycerin-monothioglykolat, Glycerin-2-mercapto-propionat, eine azeotrope Mischung aus 2-Hydroxy-propyl-thioglykolat und 2-Hydroxy-1-methyl-ethyl-thioglykolat, Cysteamin und seine N-acylierten Derivate, das Acyl-Radikal mit 2 bis 5 Kohlenstoffatomen, Cystein und die Methyl- und Ethylcysteinate.

3. Verfahren nach einem der Ansprüche 1 oder 2, dadurch gekennzeichnet, daß das Absorptionsmittel mindestens ein absorbierendes Produkt umfaßt, das aus der Gruppe ausgewählt ist, die aus Aktivkohlen, Tierkohle, Talk, Calciumcarbonat, Kieselgur, Tonen, Holzsägmehl und absorbierenden organischen Polymeren besteht.

4. Verfahren nach einem der Ansprüche 1 bis 3, dadurch gekennzeichnet, daß das Absorptionsmittel eine Korngröße von 0,1 bis 100 µm hat.

5. Verfahren nach einem der Ansprüche 1 bis 4, dadurch gekennzeichnet, daß man die flüssige Formulierung und das Absorptionsmittel durch Mischen in Kontakt bringt.

6. Verfahren nach Anspruch 5, dadurch gekennzeichnet, daß man die Formulierung 5 bis 30 Minuten vor der Verwendung der Zusammensetzung mit dem Absorptionsmittel in Kontakt bringt.

7. Verfahren nach einem der Ansprüche 1 bis 4, dadurch gekennzeichnet, daß man die flüssige Formulierung eine Absorptionsmittelschicht passieren läßt.

8. Durch das Verfahren nach einem der Ansprüche 1 bis 4 erhaltene desodorierte Zusammensetzung.

9. Desodorierte kosmetische Zusammensetzung, die dazu bestimmt ist, auf ein menschliches Keratinelement wie Haut und Haare aufgetragen zu werden, dadurch gekennzeichnet, daß sie mindestens eine Verbindung der Formel (I) nach Anspruch 1 und mindestens ein pulverförmiges Absorptionsmittel nach Anspruch 1 enthält.

10. Zusammensetzung nach Anspruuch 9, dadurch gekennzeichnet, daß die Verbindung/en der Formel (I) in einer Konzentration von 2 bis 30 Gew.-%, bezogen auf das Gesamtgewicht der Zusammetzung, vorliegt/vorliegen und das Absorptionsmittel eine Konzentration von 0,5 bis 30 Gew.-%, bezogen auf das Gesamtgewicht der Zusammensetzung, hat.

11. Zusammensetzung nach Anspruch 10, dadurch gekennzeichnet, daß die Verbindung/en der Formel (I) in ihr in einer Konzentration von 5 bis 25 Gew.-%, bezogen auf das Gesamtgewicht der Zusammensetzung, vorliegt/vorliegen und das Absorptionsmittel eine Konzentration von 1 bis 10 Gew.-%, bezogen auf das Gesamtgewicht der Zusammensetzung, hat.

12. Zusammensetzung nach einem der Ansprüche 9 bis 11, dadurch gekennzeichnet, daß ihr pH 5 bis 12,5 beträgt.

13. Zusammensetzung nach Anspruch 12, dadurch gekennzeichnet, daß sie zur Einstellung ihres pH's ein Alkalinisierungsmittel aufweist, das aus der Gruppe ausgewählt ist, die besteht aus: Ammoniak, Monoethanolamin, Diethanolamin, Triethanolamin, Alkalicarbonate, Alkalibicarbonate, Ammoniumcarbonat, Ammoniumbicarbonat und ein Alkalihydroxyd, oder ein Säuerungsmittel aufweist, das aus der Gruppe ausgewählt ist, die aus Salzsäure, Essigsäure, Milchsäure, Oxalsäure und Borsäure besteht.

14. Zusammensetzung nach einem der Ansprüche 12 oder 13, die zur bleibenden Verformung von Haaren bestimmt ist, dadurch gekennzeichnet, daß ihr pH 5 bis 10 beträgt.

15. Zusammensetzung nach einem der Ansprüche 12 oder 13, die zum Enthaaren bestimmt ist, dadurch gekennzeichnet, daß ihr pH 9 bis 12,5 beträgt.

16. Zusammensetzung nach einem der Ansprüche 9 bis 15, dadurch gekennzeichnet, daß sie eine wässrige oder hydroalkoholische Lösung mindestens einer Verbindung der Formel (I) enthält.
